(19) Europäisches Patentamt
European Patent Office
Office européen des brevets

(11) EP 4 403 542 B1

(12) EUROPEAN PATENT SPECIFICATION

(45) Date of publication and mention of the grant of the patent:
**11.02.2026 Bulletin 2026/07**

(21) Application number: **22867256.4**

(22) Date of filing: **30.08.2022**

(51) International Patent Classification (IPC):
***C07C 17/42*** (2006.01) ***C07B 63/02*** (2006.01)
***C07C 23/20*** (2006.01)

(52) Cooperative Patent Classification (CPC):
(C-Sets available)
**C07C 17/42;** C07C 2602/38 (Cont.)

(86) International application number:
**PCT/JP2022/032637**

(87) International publication number:
**WO 2023/037934 (16.03.2023 Gazette 2023/11)**

# (54) PRODUCTION METHOD FOR ENCAPSULATION OF BICYCLOALKANE COMPOUND

HERSTELLUNGSVERFAHREN ZUR VERKAPSELUNG EINER BICYCLOALKANVERBINDUNG

PROCÉDÉ DE PRODUCTION POUR L'ENCAPSULATION D'UN COMPOSÉ BICYCLOALCANE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.09.2021 JP 2021148841**

(43) Date of publication of application:
**24.07.2024 Bulletin 2024/30**

(73) Proprietor: **FUJIFILM Corporation**
**Tokyo 106-8620 (JP)**

(72) Inventors:
- **TSUNA, Kazuhiro**
  **Ashigarakami-gun, Kanagawa 258-8577 (JP)**
- **IWASAKI, Koichi**
  **Ashigarakami-gun, Kanagawa 258-8577 (JP)**
- **WADA, Kenji**
  **Ashigarakami-gun, Kanagawa 258-8577 (JP)**

(74) Representative: **HGF**
**HGF Limited**
**4th Floor, 1 City Square**
**Leeds LS1 2AL (GB)**

(56) References cited:
**CN-A- 101 120 943** **JP-A- 2010 516 766**
**JP-A- H1 129 548** **JP-A- S6 034 931**
**JP-A- S63 502 665** **JP-B1- S 503 362**
**JP-B1- S5 231 404**

- **MATSUNAGA TADAFUMI ET AL: "[alpha]-Cyclodextrin Encapsulation of Bicyclo [1.1.1]pentane Derivatives: A Storable Feedstock for Preparation of [1.1.1]Propellane", vol. 60, no. 5, 4 January 2021 (2021-01-04), Hoboken, USA, pages 2578 - 2582, XP055943070, ISSN: 1433-7851, Retrieved from the Internet <URL:https://onlinelibrary.wiley.com/doi/full-xml/10.1002/anie.202014997> [retrieved on 20250227], DOI: 10.1002/anie.202014997**
- **SU JIANYU ET AL: "Formation of [beta]-Cyclodextrin Inclusion Enhances the Stability and Aqueous Solubility of Natural Borneol", JOURNAL OF FOOD SCIENCE, vol. 77, no. 6, 14 May 2012 (2012-05-14), Hoboken, USA, XP093255191, ISSN: 0022-1147, DOI: 10.1111/j.1750-3841.2012.02713.x**
- **MIFUNE, AKIRA; SHIMA, ATSUYUKI: "Cyclodextrins and Their Application", YUKI GOSEI KAGAKU KYOKAISHI - JOURNAL OF SYNTHETIC ORGANIC CHEMISTRY, YUKI GOSEI KAGAKU KYOKAI, JP, vol. 35, no. 2, 1 February 1977 (1977-02-01), JP , pages 116 - 130, XP009544433, ISSN: 0037-9980, DOI: 10.5059/yukigoseikyokaishi.35.116**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **HIROSHI OSHIMA: "Chemistry handbook: Applied chemistry part. 7th edition", vol. 7, 1 January 2014, MARUZEN CO , JP , ISBN: 978-4-621-08759-6, article EDITED BY THE CHEMICAL SOCIETY OF JAPAN: "Passage; Handbook of Chemistry: Applied Chemistry", pages: 1580, XP009544499**
- **MATSUNAGA TADAFUMI, KANAZAWA JUNICHIRO, ICHIKAWA TOMOHIRO, HARADA MEI, NISHIYAMA YUSUKE, DUONG NGHIA TUAN, MATSUMOTO TAKASHI, MIYA: "α-Cyclodextrin Encapsulation of Bicyclo[1.1.1]pentane Derivatives: A Storable Feedstock for Preparation of [1.1.1]Propellane", ANGEWANDTE CHEMIE INTERNATIONAL EDITION, VERLAG CHEMIE, HOBOKEN, USA, vol. 60, no. 5, 1 February 2021 (2021-02-01), Hoboken, USA, pages 2578 - 2582, XP055943070, ISSN: 1433-7851, DOI: 10.1002/anie.202014997**


(52) Cooperative Patent Classification (CPC): (Cont.)

C-Sets
**C07C 17/42, C07C 23/24**

## Description

## BACKGROUND OF THE INVENTION

1. Field of the Invention

The present invention relates to a production method of an inclusion body of a bicycloalkane compound.

2. Description of the Related Art

A bicyclo[1.1.1]pentane (BCP) compound has attracted attention as a biologically active compound. A BCP motif has high three-dimensionality and high biological safety while having biological functionality equivalent to a para-substituted phenyl group, an alkynyl group, a tert-butyl group, and the like, so that it is expected to be applied to drug delivery. In addition, a halide of BCP (halogeno BCP) is a compound useful as a building block, and among these, 1,3-diiodo BCP is actually introduced in synthesis of a pharmaceutical drug candidate compound.

In synthesis of a compound having the BCP motif, a BCP compound obtained by halogenating, alkylating, acetylating, or the like BCP is used as an intermediate. It is desired to store these compounds stably as a synthetic raw material. That is, chemical stability in the air is required, and heat stability, photostability, and the like are also required. A technique for stably storing such a BCP compound has been proposed. For example, Angew. Chem. Int. Ed., 2021, vol. 60, p. 2578 to 2582 discloses that a water-insoluble inclusion body is obtained by mixing a 1,3-disubstituted BCP compound with α-cyclodextrin in water, and in the state of this inclusion body, storage stability of the 1,3-disubstituted BCP compound is greatly enhanced.

## SUMMARY OF THE INVENTION

According to the technique disclosed in Angew. Chem. Int. Ed., 2021, vol. 60, p. 2578 to 2582, it is confirmed that the inclusion body of the BCP compound can be quickly and easily obtained only by mixing the BCP compound and α-cyclodextrin in water. However, as a result of studies, the present inventors have found that, in a case where a bicycloalkane compound such as the BCP compound and a cyclodextrin compound are mixed in water, it cannot be said that efficiency of inclusion is sufficient, and particularly, in a case where an amount of preparation is increased, production efficiency of the inclusion body is significantly reduced.

An object of the present invention is to provide a production method of an inclusion body of a bicycloalkane compound, in which, in a case where a bicycloalkane compound is included a cyclodextrin compound to obtain an inclusion body of the bicycloalkane compound, yield of the obtained inclusion compound is sufficiently increased.

The object of the present invention has been achieved by the following methods.

[1] A production method of an inclusion body of a bicycloalkane compound, the production method comprising:

mixing a bicycloalkane compound with a cyclodextrin compound in a mixed solvent of water and an organic solvent; wherein

the bicycloalkane compound is a bicyclo[1.1.1]pentane compound represented by General Formula (1),

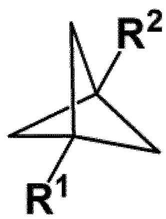

General Formula (1)

in the formula, $R^1$ and $R^2$ each independently represent a hydrogen atom or a substituent; wherein the mixing of the bicycloalkane compound and the cyclodextrin compound is carried out in a state in which light having a wavelength of 400 nm or less is shielded.

[2] The production method according to [1]
in which $R^1$ and $R^2$ each independently represent a hydrogen atom, a halogen atom, an alkyl group, an alkoxy group, an aryl group, an acyl group, a cyano group, a nitro group, an amino group, or a carboxy group.

[3] The production method according to any one of [1] to [2],
in which the cyclodextrin compound is an $\alpha$-cyclodextrin compound.
[4] The production method according to any one of [1] to [3],
in which the organic solvent is one or two or more of an ester solvent, an ether solvent, a ketone solvent, a nitrile solvent, an alcohol solvent, an amide solvent, or a hydrocarbon solvent.
[5] The production method according to any one of [1] to [4]
in which a content of the organic solvent in the mixed solvent is 80% by volume or less.
[6] The production method according to any one of [1] to [5],
in which the mixing of the bicycloalkane compound and the cyclodextrin compound in the mixed solvent of water and an organic solvent is carried out by mixing a solution obtained by dissolving the bicycloalkane compound with a solution obtained by dissolving the cyclodextrin compound.
[7] The production method according to [6],
in which the solution obtained by dissolving the cyclodextrin compound is an aqueous solution obtained by dissolving the cyclodextrin compound in water.

In the present invention or the specification, any numerical range expressed using "to" refers to a range including the numerical values before and after the "to" as a lower limit value and an upper limit value, respectively.

In the present invention, the term "... compound" means "compound having ... skeleton". For example, "bicyclo [1.1.1]pentane compound" includes not only bicyclo[1.1.1]pentane itself, but also a form in which at least a part of hydrogen atoms in the bicyclo[1.1.1]pentane is substituted.

In the present invention, a substituent which is not specified regarding whether to be substituted or unsubstituted may further have an appropriate substituent. Therefore, in the present specification, even in a case of being simply described as "... group" (for example, "alkyl group" or "amino group"), the "... group" (for example, "alkyl group") includes not only an aspect having no substituent (for example, "unsubstituted alkyl group" or "unsubstituted amino group") but also an aspect having a further substituent (for example, "substituted alkyl group" or "substituted amino group"). Similarly, "carboxy group" means to include a form of -COOR (R is a substituent), in addition to -COOH.

With the production method of an inclusion body of a bicycloalkane compound according to the aspect of the present invention, in a case where a bicycloalkane compound is included in a cyclodextrin compound to obtain an inclusion body of the bicycloalkane compound, yield of the obtained inclusion compound can be sufficiently increased.

## DESCRIPTION OF THE PREFERRED EMBODIMENTS

The production method of an inclusion body of a bicycloalkane compound according to the embodiment of the present invention (hereinafter, also referred to as "production method according to the embodiment of the present invention") includes mixing a bicycloalkane compound with a cyclodextrin compound in a mixed solvent of water and an organic solvent. By this mixing, the bicycloalkane compound is included in the cyclodextrin compound, and an inclusion body to be obtained is precipitated in the above-described mixed solvent. That is, an inclusion compound which is insoluble in the mixed solvent is obtained.

[Bicycloalkane compound]

The bicycloalkane compound used in the production method according to the embodiment of the present invention is not particularly limited as long as it is an alkane compound having a bicyclo structure. Examples thereof include a bicyclo[1.1.1]pentane (BCP) compound, a bicyclo [2.2.1]pentane (norbornane) compound, a bicyclo[4.4.0]decane (decalin) compound, and a bicyclo[3.3.1]heptane (norpinane) compound. Among these, from the viewpoint of more efficient inclusion, a BCP compound is preferable.

The above-described BCP compound is a compound represented by General Formula (1).

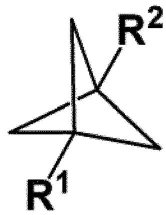

General Formula (1)

In General Formula (1), $R^1$ and $R^2$ each independently represent a hydrogen atom or a substituent. In a case where both $R^1$ and $R^2$ are substituents, $R^1$ and $R^2$ may be the same or different from each other.

The substituent which can be adopted as $R^1$ and $R^2$ is not particularly limited. From the viewpoint of more efficient inclusion, it is preferable that the number of substituents which can be adopted as $R^1$ and $R^2$ is small to some extent. For example, a group having a chemical formula weight of 400 or less is preferable, a group having a chemical formula weight of 300 or less is more preferable, a group having a chemical formula weight of 200 or less is still more preferable, and a group having a chemical formula weight of 150 or less is even more preferable.

Preferred examples of the substituent which can be adopted as $R^1$ and $R^2$ include a halogen atom (for example, a fluorine atom, a chlorine atom, a bromine atom, and an iodine atom), an alkyl group (preferably, an alkyl group having 1 to 10 carbon atoms, more preferably an alkyl group having 1 to 6 carbon atoms, still more preferably an alkyl group having 1 to 3 carbon atoms, and even more preferably methyl or trifluoromethyl), an alkoxy group (preferably an alkoxy group having 1 to 10 carbon atoms, more preferably an alkoxy group 1 to 6 carbon atoms, still more preferably an alkoxy group having 1 to 3 carbon atoms, and even more preferably methoxy), an aryl group (preferably phenyl), an acyl group (including an alkylcarbonyl group, an arylcarbonyl group, a formyl group, and a halogenocarbonyl group; the alkylcarbonyl preferably has 2 to 10 carbon atoms, more preferably has 2 to 6 carbon atoms, and still more preferably has 2 or 3 carbon atoms; the arylcarbonyl group is preferably a phenylcarbonyl group), a cyano group, a nitro group, an amino group, and a carboxy group. Among these, from the viewpoint of more efficient inclusion, a halogen atom, a cyano group, a phenyl group, or an acetyl group is preferable, and a halogen atom or an acetyl group is more preferable. The halogen atom is preferably a chlorine atom, a bromine atom, or an iodine atom, and more preferably an iodine atom.

It is also preferable that one or both of $R^1$ and $R^2$ are hydrogen atoms.

The BCP compound can be derived, for example, from [1.1.1]propellane. The [1.1.1]propellane can be obtained, for example, by reacting 1,1-dibromo-2,2-bis(chloromethyl)cyclopropane with an organometallic reagent.

As the organometallic compound, alkyllithium, aryllithium, or the like can be widely used.

The reaction itself of reacting 1,1-dibromo-2,2-bis(chloromethyl)cyclopropane with an organometallic reagent to obtain [1.1.1]propellane is already known, and for example, Chem. Commun., 2021, vol. 57, p. 2871 to 2874 can be referred to.

In addition, the BCP compound derived from [1.1.1]propellane can also be obtained by a conventional method using [1.1.1]propellane as a starting material. For example, [1.1.1]propellane and a 2,3-butanedione compound can be subjected to a photoreaction to obtain 1,3-diacetyl BCP. In addition, in a case where BCP is reacted with a halogen atom, 1,3-halogeno BCP can be obtained. These synthesis reactions themselves are known, and for example, Chem. Commun., 2021, vol. 57, p. 2871 to 2874, JP2020-533330B, and JP2019-510012B can be appropriately referred to.

[Cyclodextrin compound]

Examples of the cyclodextrin (CD) compound used in the production method according to the embodiment of the present invention include an α-CD compound, a β-CD compound, and a γ-CD compound (that is, α-CD, β-CD, γ-CD, and compounds derived from these CD's), and one kind or two or more kinds thereof can be used. Among these, an α-CD compound is preferable from the viewpoint of pore size. Examples of the α-CD compound include α-CD, mono-2-O-(p-toluenesulfonyl)-α-CD, mono-6-O-(p-toluenesulfonyl)-α-CD, and 3A-amino-3A-deoxy-(2AS,3AS)-α-CD, and one kind or two or more kinds thereof can be used.

[Mixed solvent]

In the production method according to the embodiment of the present invention, the bicycloalkane compound and the CD compound are mixed in a mixed solvent of water and an organic solvent, thereby forming an inclusion body of the bicycloalkane compound. As the organic solvent, an organic solvent capable of dissolving the bicycloalkane compound is preferable. That is, it is preferable that the CD compound can be in a dissolved state without being precipitated by at least action of water in the mixed solvent, and the bicycloalkane compound can be in a dissolved state by at least action of the organic solvent in the mixed solvent. The organic solvent may be compatible or incompatible with water. That is, water and the organic solvent may be miscible or phase-separated. It is preferable that water and the organic solvent are miscible.

Examples of the organic solvent include an ester solvent, an ether solvent, a ketone solvent, a nitrile solvent, an alcohol solvent, an amide solvent, and a hydrocarbon solvent, and one kind or two or more kinds thereof can be used.

The organic solvent is preferably at least one of an ester solvent or an ether solvent. As the ester solvent, a fatty acid ester compound is preferable. In addition, as the ether solvent, a cyclic ether compound is preferable. Among these, at least one of a tetrahydrofuran compound or a fatty acid alkyl ester compound is preferable. The number of carbon atoms in fatty acid and the number of carbon atoms in an alkyl group of the fatty acid alkyl ester are both preferably 1 to 10, more preferably 1 to 6, and still more preferably 1 to 3.

Specific examples of the ester solvent include methyl acetate, ethyl acetate, propyl acetate, phenyl acetate, methyl propionate, ethyl propionate, propyl propionate, phenyl propionate, methyl 3-methoxypropionate, methyl 3-ethoxypropionate, and methyl lactate.

Specific examples of the ether solvent include diethyl ether, ethylene glycol dimethyl ether, dibutyl ether, tetrahydrofuran, methyl cyclopentyl ether, and dioxane.

Specific examples of the ketone solvent include acetone, methyl ethyl ketone, methyl isobutyl ketone, cyclopentanone, and cyclohexanone.

Specific examples of the nitrile solvent include acetonitrile, propionitrile, and benzonitrile.

Specific examples of the alcohol solvent include methanol, ethanol, propanol, and butanol.

Specific examples of the amide solvent include N-methylpyrrolidone, 2-pyrrolidone, dimethylformamide, dimethylimidazolidinone, and dimethylacetamide.

Specific examples of the hydrocarbon solvent include butane, pentane, hexane, heptane, cyclopentane, cyclohexane, methylcyclopentane, methylcyclohexane, benzene, toluene, and xylene.

From the viewpoint of obtaining a more pure inclusion body, a content of the organic solvent in the above-described mixed solvent is preferably 80% by volume or less. The content of the organic solvent in the mixed solvent is preferably 1% to 80% by volume, more preferably 2% to 80% by volume, still more preferably 4% to 75% by volume, even more preferably 8% to 70% by volume, even still more preferably 15% to 65% by volume, further more preferably 16% to 60% by volume, even further more preferably 18% to 55% by volume, and particularly preferably 20% to 50% by volume.

[Preparation of inclusion body of bicycloalkane compound]

In the production method according to the embodiment of the present invention, it is preferable that the above-described mixing of the bicycloalkane compound and the CD compound carried out in the mixed solvent is carried out in a liquid state of the mixed solvent. For example, the mixing is carried out preferably at -5°C to 60°C, more preferably 0°C to 50°C, still more preferably 5°C to 50°C, and even more preferably 10°C to 45°C. In addition, the mixing time is not particularly limited as long as a desired amount of a target inclusion body can be obtained. For example, the bicycloalkane compound and the CD compound can be mixed for 5 to 500 minutes, preferably for 10 to 300 minutes, in a state in which the entire amounts of the bicycloalkane compound and the CD compound are mixed. In addition, the mixing time may be 5 to 60 minutes, 5 to 30 minutes, 5 to 20 minutes, or 5 to 15 minutes. Even in such a short-time mixing, the target inclusion body can be obtained in a sufficiently high yield.

As a preferred embodiment of the above-described mixing of the bicycloalkane compound and the CD compound carried out in the mixed solvent, a CD compound solution obtained by dissolving the CD compound and a bicycloalkane compound solution obtained by dissolving the bicycloalkane compound are prepared in advance, and then the CD compound solution and the bicycloalkane compound solution are mixed with each other. More preferably, a CD compound aqueous solution obtained by dissolving the CD compound in water and a bicycloalkane compound solution obtained by dissolving the bicycloalkane compound in an organic solvent are prepared in advance, and then the CD compound aqueous solution and the bicycloalkane compound solution are mixed with each other. As a result, it is possible to obtain a target inclusion body with higher efficiency.

As described above, the inclusion body of the bicycloalkane compound can be produced with high efficiency only by mixing the bicycloalkane compound and the CD compound in the above-described mixed solvent. The produced inclusion body of the bicycloalkane compound is precipitated without being dissolved in the above-described mixed solvent. The precipitated inclusion body can be recovered, for example, by a filtration treatment. The recovered inclusion body can be washed with water, an organic solvent, or the like, and as necessary, subjected to a drying treatment to isolate the inclusion body of the bicycloalkane compound.

It is also preferable that the above-described mixing of the bicycloalkane compound and the CD compound carried out in the mixed solvent is carried out in a state in which light having a wavelength of 400 nm or less is shielded. For example, in a case where the bicycloalkane compound is an iodine compound (for example, 1,3-diiodo BCP), the yield of the obtained inclusion body can be further increased by carrying out the mixing in a state in which light having a wavelength of 400 nm or less is shielded.

Hereinafter, the present invention will be more specifically described based on Examples, but the present invention is not limited to Examples.

Examples

[Example 1]

An inclusion body obtained by including 1,3-diiodo BCP in α-CD was prepared as follows.

Water (30.1 mL) and α-CD (3.04 g, 2.0 eq) were charged into a 100 mL three-necked flask, and the mixture was stirred at room temperature to dissolve the α-CD in the water, thereby obtaining an α-CD aqueous solution. In addition, 1,3-diiodo BCP (0.5 g, 1.0 eq) was dissolved in tetrahydrofuran (THF) (4.9 mL) to obtain a 1,3-diiodo BCP solution.

The 1,3-diiodo BCP solution was added dropwise to the α-CD aqueous solution in a state of shielding light of 400

nm or less and setting the temperature to 25°C. Next, the mixture was stirred for 1 hour. A proportion of water in the mixed solvent was 86% by volume, and a proportion of THF was 14% by volume. After the stirring, a precipitate was observed in the mixed solvent.

The mixed solvent including the above-described precipitate was subjected to a filtration treatment, and the precipitate was washed with water (10 mL) and then washed with THF (5 mL). The washed precipitate was dried to obtain an inclusion body in which 1,3-diiodo BCP was included in $\alpha$-CD (an inclusion body of 1,3-diiodo BCP).

From the following data, it was confirmed that the precipitate includes the inclusion body of 1,3-diiodo BCP.

Chemical shift $\sigma$ (ppm) in $^1$H-NMR (400 MHz, solvent: DMSO-d6, internal reference substance: tetramethylsilane (TMS)) = 2.71 (s, 6H), 3.25 to 3.80 (m, 72H), 4.49 to 4.51 (m, 12H), 4.79 to 4.80 (m, 12H), 5.45 to 5.46 (m, 12H), 5.52 to 5.54 (m, 12H)

The mass of the obtained precipitate was measured, and yield of crude product was calculated by the following expression.

$$\text{Yield of crude product (mol\%)} \\ = 100 \times (\text{Amount of crude product/Theoretical yield of inclusion body})$$

The purity of the inclusion body of 1,3-diiodo BCP in the precipitate was determined by an absolute calibration curve method (external standard method) using a peak surface area value calculated from high performance liquid chromatography (HPLC) under the following conditions. This purity is referred to as an LC purity.

Measuring equipment: LC-2040C 3D Plus of Shimadzu Corporation
Column: Tosoh TSKgel ODS-100Z, 5 $\mu$m, 150 $\times$ 4.6 mm
Column temperature: 40°C
Detection wavelength: 254 nm
Flow rate: 1.0 mL/min
Injection volume: 10 $\mu$L
Retention time of inclusion body: 6.95 minutes
Solvent composition: A solution: water, B solution: acetonitrile; acetic acid was added to both the A solution and the B solution to a concentration of 0.1% by volume
Gradient condition (concentration of B solution): concentration of B solution in 0 to 5.0 minutes: 20% to 100%, concentration of B solution in 5.0 to 8.0 minutes: 100%

A product of the yield (mol%) of crude product and the LC purity (%) (that is, the yield (mol%) of crude product $\times$ LC purity (%)/100) was defined as the actual yield (%), and evaluated according to the following standard.

<Evaluation standard of actual yield>

A: 60% or more
B: 50% or more and less than 60%
C: 40% or more and less than 50%
D: 30% or more and less than 40%
E: 20% or more and less than 30%
F: less than 20%

[Example 2]

An inclusion body of 1,3-diiodo BCP was obtained in the same manner as in Example 1, except that, as the organic solvent, ethyl acetate (EtOAc) was used instead of THF.

[Example 3]

An inclusion body of 1,3-diiodo BCP was obtained in the same manner as in Example 2, except that the temperatures of the dropwise addition and the stirring were set to 0°C.

[Example 4]

An inclusion body of 1,3-diiodo BCP was obtained in the same manner as in Example 2, except that the temperatures of the dropwise addition and the stirring were set to 40°C.

[Example 5]

An inclusion body of 1,3-diiodo BCP was obtained in the same manner as in Example 1, except that the light of 400 nm or less was not shielded.

[Example 6]

An inclusion body of 1,3-diiodo BCP was obtained in the same manner as in Example 2, except that the light of 400 nm or less was not shielded.

[Comparative Example 1]

An inclusion body of 1,3-diiodo BCP was obtained in the same manner as in Example 1, except that the amount of water in the α-CD aqueous solution was set to 35 mL, and instead of the dropwise addition of the 1,3-diiodo BCP solution to the α-CD aqueous solution, 1,3-diiodo BCP was added directly to the α-CD aqueous solution.

The results are shown in the following table.

[Table 1]

| | Solvent | Temperature (°C) | Shielding of light of 400 nm or less | Yield of crude product (mol%) | LC purity (%) | Actual yield |
|---|---|---|---|---|---|---|
| Example 1 | THF/water | 25 | Y | 71 | 91.1 | A |
| Example 2 | EtOAc/water | 25 | Y | 70 | 90.7 | A |
| Example 3 | EtOAc/water | 0 | Y | 77 | 78.2 | A |
| Example 4 | EtOAc/water | 40 | Y | 74 | 87.6 | A |
| Example 5 | THF/water | 25 | N | 62 | 87.8 | B |
| Example 6 | EtOAc/water | 25 | N | 63 | 87.9 | B |
| Comparative Example 1 | Water | 25 | Y | 30 | 95.0 | E |

From the results in Table 1, it was found that the actual yield of the inclusion body of 1,3-diiodo BCP was significantly increased by using the mixed solution of water and the organic solvent.

[Example 7]

An inclusion body of 1,3-diiodo BCP was obtained in the same manner as in Example 1, except that the total amount of the mixed solvent was set to 120 mL, and the proportion of THF in the mixed solvent was set to 10% by volume.

[Example 8]

An inclusion body of 1,3-diiodo BCP was obtained in the same manner as in Example 1, except that the total amount of the mixed solvent was set to 120 mL, and the proportion of THF in the mixed solvent was set to 20% by volume.

[Example 9]

An inclusion body of 1,3-diiodo BCP was obtained in the same manner as in Example 1, except that the total amount of the mixed solvent was set to 120 mL, and the proportion of THF in the mixed solvent was set to 30% by volume.

[Example 10]

An inclusion body of 1,3-diiodo BCP was obtained in the same manner as in Example 1, except that the total amount of the mixed solvent was set to 120 mL, and the proportion of THF in the mixed solvent was set to 40% by volume.

[Example 11]

An inclusion body of 1,3-diiodo BCP was obtained in the same manner as in Example 1, except that the total amount of the mixed solvent was set to 120 mL, and the proportion of THF in the mixed solvent was set to 50% by volume.

[Example 12]

An inclusion body of 1,3-diiodo BCP was obtained in the same manner as in Example 1, except that the total amount of the mixed solvent was set to 120 mL, and the proportion of THF in the mixed solvent was set to 60% by volume.

[Example 13]

An inclusion body of 1,3-diiodo BCP was obtained in the same manner as in Example 1, except that the total amount of the mixed solvent was set to 120 mL, and the proportion of THF in the mixed solvent was set to 70% by volume.

[Example 14]

An inclusion body of 1,3-diiodo BCP was obtained in the same manner as in Example 1, except that the total amount of the mixed solvent was set to 120 mL, and the proportion of THF in the mixed solvent was set to 80% by volume.

[Comparative Example 2]

An inclusion body of 1,3-diiodo BCP was obtained in the same manner as in Example 7, except that the amount of water in the α-CD aqueous solution was set to 120 mL, and instead of the dropwise addition of the 1,3-diiodo BCP solution to the α-CD aqueous solution, 1,3-diiodo BCP was added directly to the α-CD aqueous solution.

The results are shown in the following table.

[Table 2]

| | Solvent | THF concentration in solvent (% by volume) | Yield of crude product (mol%) | LC purity (%) | Actual yield |
|---|---|---|---|---|---|
| Example 7 | THF/water | 10 | 62 | 93.7 | B |
| Example 8 | THF/water | 20 | 72 | 95.1 | A |
| Example 9 | THF/water | 30 | 72 | 94.9 | A |
| Example 10 | THF/water | 40 | 75 | 94.6 | A |
| Example 11 | THF/water | 50 | 85 | 85.8 | A |
| Example 12 | THF/water | 60 | 72 | 82.2 | B |
| Example 13 | THF/water | 70 | 75 | 79.1 | B |
| Example 14 | THF/water | 80 | 73 | 71.8 | B |
| Comparative Example 2 | Water | 0 | 35 | 95.5 | D |

From the results in Table 2, it was found that, by using water and the organic solvent, the actual yield of the inclusion body of 1,3-diiodo BCP was increased regardless of the mixing ratio of the water and the organic solvent.

[Example 15]

An inclusion body of 1,3-diiodo BCP was obtained in the same manner as in Example 1, except that the total amount of the mixed solvent was set to 60 mL, the proportion of THF in the mixed solvent was set to 30% by volume, and the stirring time after the dropwise addition was set to 10 minutes.

[Example 16]

An inclusion body of 1,3-diiodo BCP was obtained in the same manner as in Example 1, except that the total amount of the mixed solvent was set to 60 mL, the proportion of THF in the mixed solvent was set to 30% by volume, and the stirring time after the dropwise addition was set to 180 minutes.

[Comparative Example 3]

An inclusion body of 1,3-diiodo BCP was obtained in the same manner as in Example 15, except that the amount of water in the α-CD aqueous solution was set to 60 mL, and instead of the dropwise addition of the 1,3-diiodo BCP solution to the α-CD aqueous solution, 1,3-diiodo BCP was added directly to the α-CD aqueous solution.

[Comparative Example 4]

An inclusion body of 1,3-diiodo BCP was obtained in the same manner as in Example 16, except that the amount of water in the α-CD aqueous solution was set to 60 mL, and instead of the dropwise addition of the 1,3-diiodo BCP solution to the α-CD aqueous solution, 1,3-diiodo BCP was added directly to the α-CD aqueous solution.

The results are shown in the following table.

[Table 3]

| | Solvent | Stirring time (min) | Yield of crude product (mol%) | LC purity (%) | Actual yield |
|---|---|---|---|---|---|
| Example 15 | THF/water | 10 | 79 | 98.5 | A |
| Example 16 | THF/water | 180 | 82 | 98.9 | A |
| Comparative Example 3 | Water | 10 | 17 | 95.3 | F |
| Comparative Example 4 | Water | 180 | 51 | 95.8 | C |

From the results in Table 3, it was found that, by using water and the organic solvent, it was possible to increase the actual yield of high-purity inclusion body of 1,3-diiodo BCP with a sufficiently high yield in a short time.

The present application claims the priority of JP2021-148841 filed in Japan on September 13, 2021.

## Claims

1. A production method of an inclusion body of a bicycloalkane compound, the production method comprising:

   mixing a bicycloalkane compound with a cyclodextrin compound in a mixed solvent of water and an organic solvent;
   wherein the bicycloalkane compound is a bicyclo[1.1.1]pentane compound represented by General Formula (1),

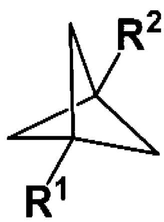

General Formula (1)

   in the formula, $R^1$ and $R^2$ each independently represent a hydrogen atom or a substituent;
   wherein the mixing of the bicycloalkane compound and the cyclodextrin compound is carried out in a state in which light having a wavelength of 400 nm or less is shielded.

2. The production method according to claim 1,
   wherein $R^1$ and $R^2$ each independently represent a hydrogen atom, a halogen atom, an alkyl group, an alkoxy group, an aryl group, an acyl group, a cyano group, a nitro group, an amino group, or a carboxy group.

3. The production method according to any one of claims 1 or 2, wherein the cyclodextrin compound is an α-cyclodextrin compound.

4. The production method according to any one of claims 1 to 3, wherein the organic solvent is one or two or more of an ester solvent, an ether solvent, a ketone solvent, a nitrile solvent, an alcohol solvent, an amide solvent, or a hydrocarbon solvent.

5. The production method according to any one of claims 1 to 4, wherein a content of the organic solvent in the mixed solvent is 80% by volume or less.

6. The production method according to any one of claims 1 to 5, wherein the mixing of the bicycloalkane compound and the cyclodextrin compound in the mixed solvent of water and an organic solvent is carried out by mixing a solution obtained by dissolving the bicycloalkane compound with a solution obtained by dissolving the cyclodextrin compound.

7. The production method according to claim 6, wherein the solution obtained by dissolving the cyclodextrin compound is an aqueous solution obtained by dissolving the cyclodextrin compound in water.

**Patentansprüche**

1. Produktionsverfahren eines Einschlusskörpers einer Bicycloalkanverbindung, wobei das Produktionsverfahren umfasst:

   Mischen einer Bicycloalkanverbindung mit einer Cyclodextrinverbindung in einem gemischten Lösungsmittel aus Wasser und einem organischen Lösungsmittel;
   wobei die Bicycloalkanverbindung eine Bicyclo[1.1.1]pentanverbindung ist, die durch allgemeine Formel (1) dargestellt wird,

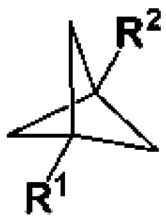

Allgemeine Formel (1)

   in der Formel $R^1$ und $R^2$ jeweils unabhängig voneinander ein Wasserstoffatom oder einen Substituenten darstellen;
   wobei das Mischen der Bicycloalkanverbindung und der Cyclodextrinverbindung in einem Zustand, in dem Licht mit einer Wellenlänge von 400 nm oder weniger abgeschirmt ist, durchgeführt wird.

2. Produktionsverfahren nach Anspruch 1, wobei $R^1$ und $R^2$ jeweils unabhängig voneinander ein Wasserstoffatom, ein Halogenatom, eine Alkylgruppe, eine Alkoxygruppe, eine Arylgruppe, eine Acylgruppe, eine Cyanogruppe, eine Nitrogruppe, eine Aminogruppe oder eine Carboxygruppe darstellen.

3. Produktionsverfahren nach einem der Ansprüche 1 oder 2, wobei die Cyclodextrinverbindung eine α-Cyclodextrinverbindung ist.

4. Produktionsverfahren nach einem der Ansprüche 1 bis 3, wobei das organische Lösungsmittel eines oder zwei oder mehr von einem Esterlösungsmittel, einem Etherlösungsmittel, einem Ketonlösungsmittel, einem Nitrillösungsmittel, einem Alkohollösungsmittel, einem Amidlösungsmittel oder einem Kohlenwasserstofflösungsmittel ist.

5. Produktionsverfahren nach einem der Ansprüche 1 bis 4, wobei ein Gehalt des organischen Lösungsmittels in dem gemischten Lösungsmittel 80 Volumen-% oder weniger

beträgt.

6. Produktionsverfahren nach einem der Ansprüche 1 bis 5,
wobei das Mischen der Bicycloalkanverbindung und der Cyclodextrinverbindung in dem gemischten Lösungsmittel aus Wasser und einem organischen Lösungsmittel durch Mischen einer Lösung, die durch Auflösen der Bicycloalkanverbindung erhalten wird, mit einer Lösung, die durch Auflösen der Cyclodextrinverbindung erhalten wird, durchgeführt wird.

7. Produktionsverfahren nach Anspruch 6,
wobei die Lösung, die durch Auflösen der Cyclodextrinverbindung erhalten wird, eine wässrige Lösung ist, die durch Auflösen der Cyclodextrinverbindung in Wasser erhalten wird.

## Revendications

1. Procédé de production d'un corps d'inclusion d'un composé de bicycloalcane, le procédé de production comprenant :

mélanger un composé de bicycloalcane avec un composé de cyclodextrine dans un solvant mixte d'eau et d'un solvant organique ;
dans lequel le composé de bicycloalcane est un composé de bicyclo[1.1.1]pentane représenté par Formule générale (1),

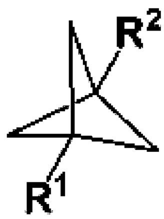

Formule générale (1)

dans la formule, $R^1$ et $R^2$ représentent chacun indépendamment un atome d'hydrogène ou un substituant ;
dans lequel le mélange du composé de bicycloalcane et du composé de cyclodextrine est effectué dans un état où la lumière ayant une longueur d'onde de 400 nm ou moins est protégée.

2. Procédé de production selon la revendication 1,
dans lequel $R^1$ et $R^2$ représentent chacun indépendamment un atome d'hydrogène, un atome d'halogène, un groupe alkyle, un groupe alcoxy, un groupe aryle, un groupe acyle, un groupe cyano, un groupe nitro, un groupe amino ou un groupe carboxy.

3. Procédé de production selon l'une quelconque des revendications 1 ou 2,
dans lequel le composé de cyclodextrine est un composé de $\alpha$-cyclodextrine.

4. Procédé de production selon l'une quelconque des revendications 1 à 3,
dans lequel le solvant organique est un ou deux ou plusieurs parmi un solvant d'ester, un solvant d'éther, un solvant de cétone, un solvant de nitrile, un solvant d'alcool, un solvant d'amide ou un solvant d'hydrocarbure.

5. Procédé de production selon l'une quelconque des revendications 1 à 4,
dans lequel une teneur en solvant organique dans le solvant mixte est de 80 % en volume ou moins.

6. Procédé de production selon l'une quelconque des revendications 1 à 5,
dans lequel le mélange du composé de bicycloalcane et du composé de cyclodextrine dans le solvant mixte d'eau et d'un solvant organique est effectué en mélangeant une solution obtenue en dissolvant le composé de bicycloalcane avec une solution obtenue en dissolvant le composé de cyclodextrine.

7. Procédé de production selon la revendication 6,
dans lequel la solution obtenue en dissolvant le composé de cyclodextrine est une solution aqueuse obtenue en dissolvant le composé de cyclodextrine dans l'eau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020533330 B
- JP 2019510012 B
- JP 2021148841 A


### Non-patent literature cited in the description


- *Angew. Chem. Int. Ed.*, 2021, vol. 60, 2578-2582
- *Chem. Commun.*, 2021, vol. 57, 2871-2874